# EUROPEAN PATENT APPLICATION

(11) **EP 1 208 751 A1**
(43) Date of publication of application: **29.05.2002**
(21) Application number: 00956846.0
(22) Date of filing: 31.08.2000
(51) Int. Cl.: A23K 1/18, A23K 1/16, A61K 31/085, A61K 31/343, A61K 31/352, A61K 31/045, A61K 31/22, A61P 33/00

(54) **NATURAL PHYSIOLOGICALLY ACTIVE SUBSTANCES EFFICACIOUS AGAINST FISH PARASITES AND FISH FEEDS CONTAINING THE SUBSTANCES**

(30) Priority: 03.09.1999 JP 24944699
(71) Applicant: Nippon Suisan Kaisha, Ltd., Tokyo 100-0004 (JP)
(72) Inventor: HIRAZAWA, Noritaka, Nippon Suisan Kaisha, Ltd., Minamikaifu-gun, Oita 876-120 (JP); HIRATA, Tatsuyoshi, Nippon Suisan Kaisha, Ltd., Minamikaifu-gun, Oita 876-120 (JP); OTAKA, Taro, Nippon Suisan Kaisha, Ltd., Minamikaifu-gun, Oita 876-1204 (JP); HATA, Kazuhiko, Nippon Suisan Kaisha, Ltd., Minamikaifu-gun, Oita 876-1204 (JP)
(74) Representative: Leson, Thomas Johannes Alois, Dipl.-Ing.
(86) International application number: JP0005923
(87) International publication number: WO0117367

(57) **Abstract**

Providing a naturally occurring physiologically active substance effective for fish parasites, and a fish feed containing the substance, wherein the naturally occurring physiologically active substance is an essential oil containing eugenol, cineol, citronellal, menthol and/or linalyl acetate and has an effect on fish parasitism and wherein the fish feed contains the essential oils as naturally occurring physiologically active substances; and also providing a fish feed containing essential oils containing eugenol, cineol, citronellal, menthol and/or linalyl acetate as a naturally occurring physiologically active substance with an effect on fish parasitism.

## Description

### Technical Field

The present invention relates to a naturally occurring physiologically active substance effective for fish parasites and a fish feed containing the same.

### Background of the Invention

Because parasitism blocks stable fish culture production, parasitism is a very serious problem. For example, Heterobothrium okamotoi infection is a serious problem in Tiger puffer (Tahifugu rubripes) cultivation. The life cycle of the parasite comprises a step for first allowing a oncomiracidia hatched from an egg to be parasitic to the gill, where the oncomiracidia is grown into a larvae of parasite to about 5 to 6 mm, and a step of transfer of the larvae of parasite to tissues surrounding the gill, into which the larvae of parasite embeds the grasping organ side and in which the larvae of parasite is grown to an adult parasite for laying egg. The effect due to periodic immersion of the fish species in formalin around 800 ppm is only an effect available for the parasite on site. However, the process can rid only about 80 % of the larvae of parasites but never can rid the adult parasite. Additionally, formalin is a carcinogenic substance and is therefore problematic from the respect of environmental pollution and the like. Thus, it is essential to develop a naturally occurring substance with an effect to prevent and rid the parasite and other parasites when orally given to the fish suffering from them. Fish culture damages with parasites are so severe that appropriate ridding thereof is desired.

### Disclosure of the Invention

It is a purpose of the invention to make investigations about a naturally occurring physiologically active substance effective for fishes, with no damage on the fishes but with an action only on parasites. It is a purpose of the invention to provide a naturally occurring physiologically active substance effective for fish parasites and a fish feed containing the substance.

So as to find a naturally occurring substance with an effect on fish parasites when given orally, the inventors have made screenings about various naturally occurring substances. Consequently, the inventors have found that eugenol, cineol, citronellal, menthol and linalyl acetate contained in essential oils are effective for fish parasites and can prevent and cure parasitism when the substances are given orally.

Thus, the invention is summarized as a naturally occurring physiologically active substance with an effect on fish parasitism, which is an essential oil containing eugenol, cineol, citronellal, menthol and/or linalyl acetate.

Additionally, the invention is summarized as a fish feed containing essential oils containing eugenol, cineol, citronellal, menthol and/or linalyl acetate as naturally occurring physiologically active substances.

Still additionally, the invention is summarized as a fish feed containing essential oils containing eugenol, cineol, citronellal, menthol and/or linalyl acetate as naturally occurring physiologically active substances with an effect on fish parasitism.

### Brief Description of the Drawings

Fig. 1 depicts graphs expressing the results of the comparison of the survival of infected fishes (n = 6);
Fig. 2 depicts graphs expressing the results of the comparison of the survival of normal fishes (n = 8);
Fig. 3 depicts bar graphs expressing the number of the parasites in infected fishes surviving at the termination of the test; and
Fig. 4 depicts bar graphs expressing the results of the comparison of the number of the parasites in normal fishes (n = 4) on day 30.

### Detailed Description of the Invention

In the same manner as in the following examples, it can be verified that the naturally occurring physiologically active substance of the invention is effective on fishes and is a substance exerting an action only on parasites with no damage on fishes and the substance is effective for fish parasitism and the appropriate concentration thereof and the like can be confirmed as well. Any of fish species with possible damages with fish ectoparasites during fish culture, which are desirably ridded properly, is suitable and includes for example cultured fishes such as salmon, red salmon, king salmon, rainbow trout, silver salmon, eel, sweetfish, yellowtail, amberjack, Tiger puffer, red sea bream and hardtail.

Any fish parasitism occurring due to fish parasites emerging on site in fish culture and of which proper ridding is desired is the subject of the invention. For example, Heterobothriosis of Tiger puffer, benedeniasis of yellowtail, hiramasa, amberjack and longfin amberjack, bibagina disease of red sea bream, bibagina disease of yellowtail, futagomushi disease of carp and crucian carp, Caligus disease of salmon family fishes and hardtail are listed.

The essential oils for use in accordance with the invention are volatile components recovered by steam distillation and the like from plant leaves, flowers, seeds, bark, fruits and fruit peel. Additionally, eugenol is the principal component contained in clove oil or cinnamon oil, while cineol also referred to eucalyptol is the principal component contained in eucalyptus oil; menthol is the principal component contained in mint oil; linalyl acetate is the principal component of lavender oil. Additionally, citronellal is one component contained in eucalyptus oil.

When the essential oils are added in appropriate forms such as solution, granule and tablet in accordance with the invention, the essential oils are preferably contained at a concentration of 0.0001 to 5 % by weight in the feed. The essential oils are introduced at a technically appropriate stage of the feed production so that the essential oils can be uniformly distributed in the final product.

The naturally occurring physiologically active substance effective for fish parasites, in accordance with the invention, can be used at any form, including, with no specific limitation, the use as chemical immersion solutions with anti-parasite actions and the use as fish feeds. The composition or production process of the inventive fish feed is not specifically limited. Any feed with addition of essential oils containing eugenol, cineol, citronellal, menthol and linalyl acetate may be satisfactory.

### Best Mode for Carrying out the Invention

The details of the invention is described in examples. The invention is not limited to these examples.

The fundamental composition of a feed used at tests is as follows.
(1) Blend of raw materials
   50 % by weight of brown meal; 17 % by weight of defatted rice bran; 25 % by weight of defatted soy bean; 5 % by weight of wheat flour; and 3 % by weight of vitamin and minerals.
(2) Feed components
   9.8 % by weight of water; 49.1 % crude protein; 5.0 % by weight of crude fat; 18.7 % by weight of soluble substance with no nitrogen; 3.1 % by weight of crude fiber; and 14.3 % by weight of crude ash.

### Examples

### <Purpose>

When larvae of parasites Heterobothrium okamotoi were immersed in sea water in which clove oil was dissolved, a phenomenon that the parasites were shrunk and led to death was observed. The *in vitro* effect of various essential oils on the ridding of Heterobothrium okamotoi problematic for Tiger puffer was tested at test 1; and at test 2, the same effect thereof on Caligus problematic for Tiger puffer, hardtail and salmons was also tested. At test 3, furthermore, it was examined whether or not the essential oils added to a feed for Tiger puffer so as to administer the essential oils to Tiger puffer could prevent Heterobothriosis and cure the disease.

### Test 1

### <Method>

Eucalyptus oil, clove oil, cinnamon oil, lavender oil and mint oil were individually dissolved in sea water to a concentration of 800 ppm, while eugenol, cineol, citronellal, menthol and linalyl acetate were individually dissolved therein to a concentration of 500 ppm; then, larvae of parasites Heterobothrium okamotoi at a parasitic state on gill were immersed in the resulting solutions. The parasite bodies were observed periodically, to judge the effects. 30 or more larvae of parasites were used per each lot. Additionally, one lot was for simple immersion in sea water, which was used as a control lot.

### <Results and discussion>

It was observed that all the essential oils and all the components of the essential oils caused shrinking of the parasites. Further, the shrinking was observed in all the lots within 5 minutes, so that 100 % of the shrunk parasites were dissociated from the gill. Alternatively, no change was observed in the parasites of the control lot, with no dissociation of the parasites from the gill. The results indicate that the essential oils have an eradication effect on Heterobothrium okamotoi.

### Test 2

### <Method>

The adults Caligus were collected from the body surface of trout. Then, eight of the parasites were divided in each petri dish. Solutions in sea water of eucalyptus oil, clove oil, cinnamon oil, lavender oil, mint oil, cineol, eugenol, citronellal, menthol and linalyl acetate adjusted to a concentration of 400 ppm were added to the petri dishes. The effects were judged under observation of the parasites over time. Additionally, a lot to which sea water was added was used as a control lot.

### <Results and discussion>

All the essential oils and all the principal components thereof were observed to exert an action to terminate the action of the parasite. Additionally, the phenomenon was observed in all within 15 minutes. Alternatively, no change was observed in the parasites of the control lot. The results indicate that the essential oils have an eradication effect on Caligus. Additionally, the effect was also examined at the stage of larvae of parasites Caligus. At a concentration of 100 ppm, the action was terminated within 6 minutes, which indicates that the larvae of parasites Caligus were more sensitive than the adult Caligus. Because Heterobothrium okamotoi belongs to flatworms and Caligus belongs to arthropods, it was indicated that the essential oils had an eradication effect on a wide variety of fish parasites species.

### Test 3

### <Method>

Test fish: Tiger puffer of a mean body weight of about 133.6 g were used at the test.
Test lots: Three lots in total were tested, namely eucalyptus addition lot to which a eucalyptus oil-added feed was fed, a clove oil addition lot to which a clove oil-added feed was fed, and a control lot to which a feed without any additive was fed.
Test feeds: essential oil-added feeds were prepared by spraying 2.5 g of an essential oil over 1 kg of a commercially available Tiger puffer pellet. To the control lot, the commercially available Tiger puffer pellet was fed.

### <Attack test>

12 fishes of a group with no observed diseases such as Heterobothrium okamotoi infection (normal fishes) were individually placed in a 100-liter water tank, to which were fed with the individual test feeds. After seven days, 6 fishes preliminarily infected with about 100 adult Heterobothrium okamotoi parasites (infected fishes) were added to each lot, for continuous feeding with the test feeds for 73 days. 30 days after the addition of infected fishes , 4 normal fishes were sampled from each lot, to count the parasites parasitic to the gill. The temperature of water during the test period was 16.5 to 20.9 °C. Water exchange during the test period was at 20 cycles/day.

Assessment was made, on comparison of the death status in each lot during the test period and comparison of the number of the parasites counted on the gills of the test fishes in each lot so as to examine the curing effect and the effect on the prevention of the infection.

### <Results and discussion>

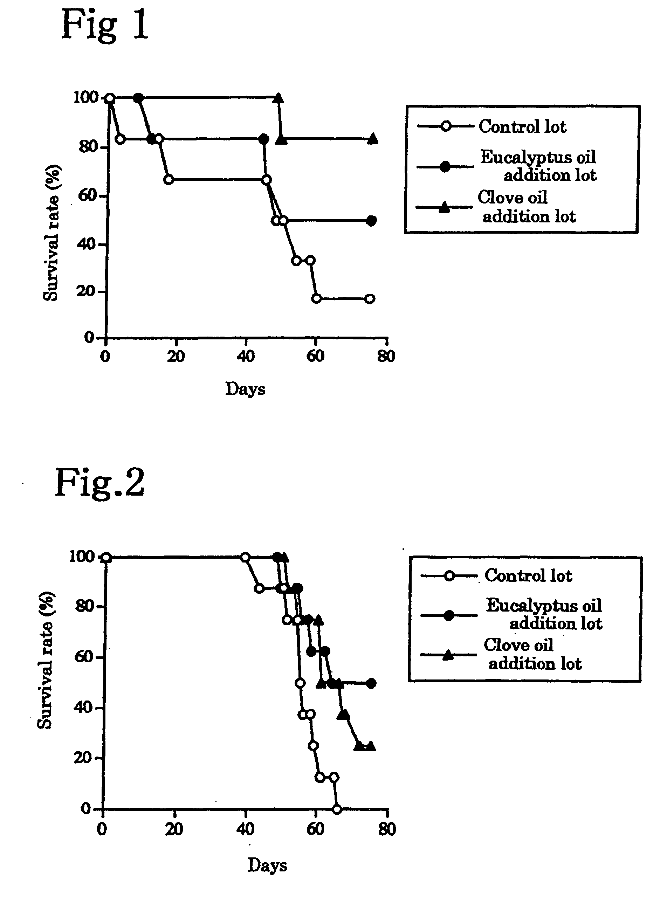

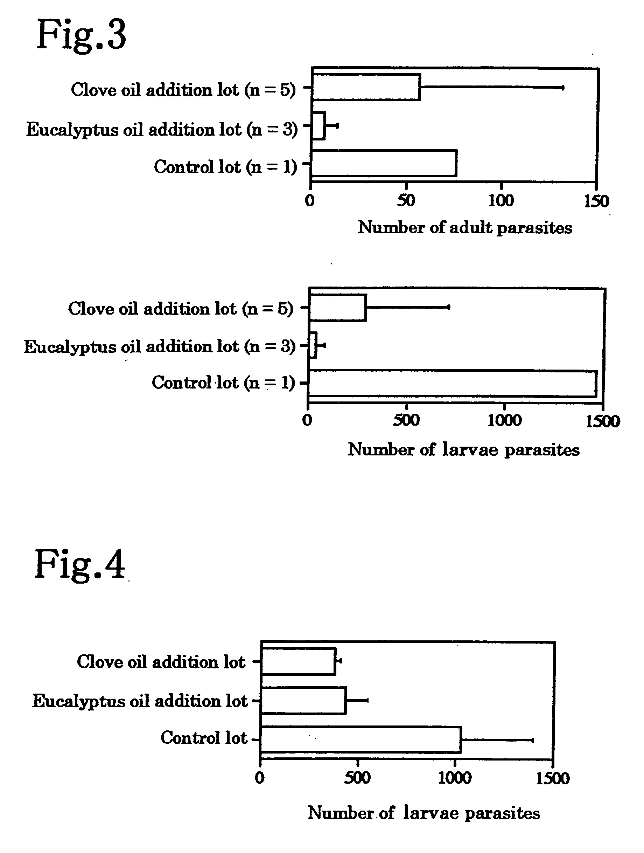

The survival rates of the infected fishes at the termination of the test were 50.0 % in the eucalyptus oil addition lot, 83.3 % in the clove oil addition lot and 16.7 % in the control lot, which indicates the effect of oral administration of the essential oils (Fig. 1). Almost no parasitism with the present adult or larvae of parasites were observed in the infected fishes surviving in the eucalyptus oil addition lot (Fig. 3). As to the status of the fishes infected with the parasite in the clove oil addition feed lot, the parasitism in the infected fishes was apparently likely to be at a lower degree, compared with the control. About 100 adult parasites parasitic to the infected fishes in the essential oil addition lots at the start of the test were decreased apparently at the termination of the test. The results indicate that both the essential oils administerd orally have an effect to rid the adult parasite, namely the effect to cure the parasitism. It is not considered that chemical agents such as hydrogen peroxide and formalin have an effect to rid the adult parasites, so the chemical agents can never prevent the proliferation over the next generation. Thus, hydrogen peroxide and the like when used can never completely eradicate the infection of the parasite to cure the parasitism, unless these are used for treatment in a repetitive manner.

Alternatively, the survival of the normal fishes was 50.0 % in eucalyptus oil addition lot, 12.5 % in the clove oil addition lot and 0 % in the control lot, which indicates the same effect as described above (Fig. 2). Compared with the control, the numbers of the parasites in the essential oil addition lots on day 30 were apparently smaller, which indicates the same effect as described above (Fig. 4). The numbers of the parasites on average in the surviving fishes in the eucalyptus oil addition lot and the clove oil addition lot at the termination of the test were 260 and 600, respectively. On contrast, the parasites parasitic to the dead fishes in the control lot were 2400 on average, while the fishes in the control lot were all dead.

Based on the results, it is verified that eucalyptus oil and clove oil added to feeds for oral dosing to fishes exert the effect on the prevention of Heterobothriosis and the curing effect in the infected fishes. Additionally, it is expected that essential oils added to feeds can exert an effect to eradicate parasites parasitic to fishes.

### Industrial Applicability

A naturally occurring physiologically active substance with an effect on fish parasitism can be provided. A fish feed containing a naturally occurring physiologically active substance with an effect on the parasitism in fishes can be provided.

Fishes can be cultured, with no death due to fish parasitism, by simply using the naturally occurring physiologically active substance as a feed component or as an immersion solution with an anti-parasitic action.

## Claims

1. A naturally occurring physiologically active substance being an essential oil containing one or more components selected from the group consisting of eugenol, cineol, citronellal, menthol and linalyl acetate and having an effect on fish parasitism.

2. A fish feed containing an essential oil containing one or more components selected from the group consisting of eugenol, cineol, citronellal, menthol and linalyl acetate as a naturally occurring physiologically active substance.

3. A fish feed containing the essential oils comprising the components as naturally occurring physiologically active substances with an effect on fish parasitism.
